# EUROPEAN PATENT APPLICATION

(11) **EP 4 400 049 A1**
(43) Date of publication of application: **17.07.2024**
(21) Application number: 23305036.8
(22) Date of filing: 11.01.2023
(51) Int. Cl.: A61B 5/256, A61B 5/27, A61B 5/00

(54) **ASSEMBLY OF A FLEXIBLE ELECTRODE AND A CONDUCTIVE YARN AND WEARABLE ARTICLE INCLUDING THE ASSEMBLY**

(71) Applicant: CHRONOLIFE, 75012 Paris (FR)
(72) Inventor: Roger, Harmony, 77173 CHEVRY COSSIGNY (FR)
(74) Representative: Plasseraud IP

(57) **Abstract**

The disclosure refers to an electrode assembly (101), comprising:
- a flexible electrode (102) having a front face (108) and a back face (104), the front face (108) comprising at least one contact area (103) to be applied to a body of a user and a border area (111) surrounding the at least one contact area (103);
- a first yarn (106) sewn in the border area (111) of the flexible electrode (102) and forming loops (107) on at least the back face (104); and
- a conductive yarn (105) passing through at least one of the loops (107) on the back face and maintained on the flexible electrode (102) by the first yarn (106).

## Description

The present disclosure generally relates to the field of assemblies of physiological sensors and conductive yarn and, in particular, relates to an assembly of a flexible electrode and a conductive yarn, and to a wearable article including such an assembly.

### BACKGROUND OF THE INVENTION

Electronic devices for detecting and processing biometric signals of a user have been developed that allow medical supervision of people during their usual activities or situations. Sensors may be applied to the skin or a specific body area, for example the chest, of a user so as to perform temperature measurements, to determine physiological parameters or to detect electrical signals related for example to a cardiac function. Monitoring the various signals delivered by these sensors allows determining a specific physiological condition of a user that might be impaired. For example, when an individual is having a seizure, specific signal features appear on the signals corresponding to the electrocardiogram (ECG) or to respiration.

A convenient possibility to permanently monitor cardiac activity of a user is to integrate electrodes into a garment or some other wearable article. Such electrodes may acquire information about parameters such as frequency and amplitude of cardiac activity of a user and transmit it to external medical equipment, such as monitoring, diagnostic, or stimulating devices.

Conductive yarn may be used for electrically connecting the electrodes to other electronic equipment in the garment. Such conductive yarn may be sewn through the flexible body of the electrode in order to create an electrical connection between the electrode and the conductive yarn as described in WO 2021/048211 A1.

However, conductive yarn is often fragile and may be degraded when sewing it through the body of the electrode.

Furthermore, conductive yarn and flexible electrodes are often fabricated from materials such as silicone that do not adhere to other materials, which generally complicates connection of conductive yarn to electrodes.

### SUMMARY OF THE INVENTION

An electrode assembly is disclosed, comprising:
- a flexible electrode having a front face and a back face, the front face comprising at least one contact area to be applied to a body of a user and a border area surrounding the at least one contact area;
- a first yarn sewn in the border area of the flexible electrode and forming loops on at least the back face; and
- a conductive yarn passing through at least one of the loops on the back face and maintained on the flexible electrode by the first yarn.

The loops of the first yarn may press the conductive yarn against the back face of the flexible electrode and tie the conductive yarn to the electrode, making the assembly of the flexible electrode and the conductive yarn stable and robust.

The at least one contact area may be a portion of the flexible electrode intended to be in contact with the body of the user and to establish an electrical contact between the body of the user and the flexible electrode.

In some embodiments, the at least one contact area may protrude from the border area on the front face.

One or more contact areas may protrude from the front face, i.e. each of said one or more contact areas may comprise a protrusion. This may allow optimizing the electrical contact between the body of the user and the flexible electrode.

In some embodiments, the electrode assembly may comprise a plurality of contact areas, i.e. a plurality of protrusions protruding from the front face.

In some embodiments, each protrusion can be of any shape, such as needle, column, cones, pillar, cylinder, frustum of a cone, prism having a polygonal cross section, regular or irregular pyramid, and combination thereof. In some embodiments, one or more protrusions may be substantially cylindrical.

In some embodiments, the average height of the at least one protrusion may be greater than about 1 mm, such as from about 1 to about 5 mm, preferably from about 1 to about 4 mm, more preferably from about 1 to about 3 mm. In some embodiments, the average height of the at least one protrusion may be about 2 mm.

The term "about" as used herein means within 20%, preferably within 10%, and more preferably within 5%. In specific case, "about X", means "X".

In some embodiments, each protrusion may have a diameter in a range of about 2 to about 5 mm, preferably from about 2 to about 4 mm, more preferably from about 2 to about 3 mm. In some embodiments, the diameter of each protrusion may be about 3 mm.

In some embodiments, the first yarn sewn in the border area of the flexible electrode may form loops on the back and front faces.

In one embodiment, the body of the flexible electrode (i.e. the distance from the front to the back face) may have a thickness in a range of about 1 to about 5 mm, preferably from about 1 to about 4 mm, more preferably from about 1 to about 3 mm. In some embodiments, the flexible electrode thickness may be about 1 mm.

In an embodiment, the electrode assembly further may comprise a protective fabric disposed on the border area of the front face and exposing the at least one contact area.

The protective fabric may protect the flexible electrode against humidity and wear.

In an embodiment, the electrode assembly may further comprise a second yarn sewn through the protective fabric and the border area of the front face.

The second yarn may attach the protective fabric to the flexible electrode.

In an embodiment, the flexible electrode may comprise conductive particles embedded in flexible material.

The choice of the flexible material may allow tailoring the mechanical properties of the flexible electrode. The conductivity of the flexible electrode may be tailored by the material used for the conductive particles and by the concentration and the arrangement of the conductive particles in the flexible body.

In an embodiment, the first yarn may comprise polyester.

A polyester yarn may be easily manipulated. The polyester yarn may establish a stable connection between the conductive yarn and the flexible electrode.

In an embodiment, the conductive yarn may comprise an arrangement of chain stitches.

Compared to a conductive yarn in a linear arrangement, the arrangement of a conductive yarn in chain stitches may be more resilient, may be more easily manipulated, and may adhere better to the back face of the flexible electrode.

In an embodiment, the conductive yarn may be sewn to a flexible substrate.

The flexible substrate may be made from a flexible material and provide a support for the conductive yarn. A conductive yarn sewn to a flexible substrate may be more stable and resilient when forces are applied to the conductive yarn.

In an embodiment, the conductive yarn may have a connection portion extending beyond the flexible substrate. The connection portion may pass through at least some of the loops. The connection portion may be maintained on the flexible electrode by the first yarn.

The connection portion may be an end of the conductive yarn or be located close to an end of the conductive yarn.

The electrical contact between the flexible electrode and the conductive yarn may be optimized when using the connection portion for creating the electrical contact rather than another portion of the conductive yarn, in particular if the flexible substrate is bulky or has non-conductive properties.

Another aspect of the disclosure is related to a wearable article, comprising:
- an electronic board;
- a flexible electrode and a conductive yarn forming an electrode assembly as described above, wherein the conductive yarn may further be connected to the electronic board.

The wearable article may be configured to press the at least one contact area of the flexible electrode against the body of the user wearing the wearable article.

The electronic board may be configured to detect variations of electrically conductive properties of the flexible electrode. For example, information related to heart activity or to breathing may be deduced from these variations.

In an embodiment, the wearable article may further comprise an electromagnetic shielding material located at the back face of the flexible electrode. An insulating material may be placed between the back face of the flexible electrode and the electromagnetic shielding material.

The electromagnetic shielding material may shield the flexible electrode from external electromagnetic perturbations, coming for example from electronic devices located in the vicinity of the flexible electrode during measurements with the flexible electrode.

In an embodiment, the wearable article further may comprise an elastic belt on which the flexible electrode is mounted. The elastic belt may be configured to press the at least one contact area of the flexible electrode against the body of the user wearing the wearable article.

The elastic belt may allow attaching the wearable article to the body of the user.

In an embodiment, the wearable article may further comprise a waterproof housing configured to receive the electronic board. The elastic belt may be attached to the waterproof housing.

The waterproof housing may protect the electronic board against humidity and other kinds of perturbations.

In an embodiment, the wearable article may be in the form of a garment. The wearable article may further comprise a flexible support connected to the waterproof housing.

The garment may refer to an item of clothing or apparel. The garment may be a top. The top may be a shirt, t-shirt, blouse, sweater, dress, underwear, athletic clothing, swimwear, belt, jacket/coat, or vest.

The flexible support may be stretched when worn by the user and press the flexible electrode against the body of the user.

In an embodiment, the waterproof housing may comprise an orifice. The conductive yarn maintained on the flexible electrode and connected to the electronic board may pass through said orifice.

Thus, the electronic board located inside the waterproof housing may be connected to the flexible electrode located outside the waterproof housing by the conductive yarn.

In an embodiment, the orifice may be sealed by elastic tape.

The elastic tape may be placed on the orifice in order to ensure that the space enclosed by the polyester plates is waterproof.

In an embodiment, the wearable article may further comprise a T-Shirt having a flexible support configured to press the at least one contact area of the flexible electrode against the body of the user wearing the wearable article.

The integration of flexible electrodes into the T-Shirt may allow studying parameters related to breathing or to heart activity over many hours, as long as the T-Shirt is worn.

### BRIEF DESCRIPTION OF THE DRAWINGS

The accompanying drawings, which are incorporated herein and form a part of the specification, illustrate the present invention and, together with the description, further serve to explain the features of the invention and to enable a person skilled in the pertinent art to make and use the invention. In the drawings, like reference characters indicate identical or functionally similar elements.
- Figure 1 is a schematic representation of the front side of an example of a wearable article according to the invention, the wearable article being a brassiere comprising electrode assemblies;
- Figure 2 is a schematic representation of the front side of another example of a wearable article according to the invention, the wearable article being a T-Shirt comprising electrode assemblies;
- Figure 3 is a schematic representation of the back side of the T-shirt of Fig. 2;
- Figure 4 is a schematic representation of an embodiment of an electrode assembly in a perspective view;
- Figure 5 is a side view of the electrode assembly of Fig. 4;
- Figure 6 is a schematic representation of a conductive yarn sewn to a flexible support, the conductive yarn being part of the electrode assembly;
- Figure 7 is a perspective view of an electrode assembly according to one embodiment of the invention;
- Figure 8 is a top view of a electrode assembly according to one embodiment of the invention;
- Figure 9 is a side view of said electrode assembly according to one embodiment, from direction A - A shown in Figure 8.
- Figure 10 is a side view of said electrode assembly according to one embodiment, from direction A - A shown in Figure 8.

### DESCRIPTION OF PREFERRED EMBODIMENTS

The electrode assembly of the invention can be comprised in a wearable article that includes physiological sensors for detecting physiological signals of the body of a user wearing the wearable article, also referred to as wearer. At least one of said sensors is a flexible electrode of an electrode assembly according to the invention.

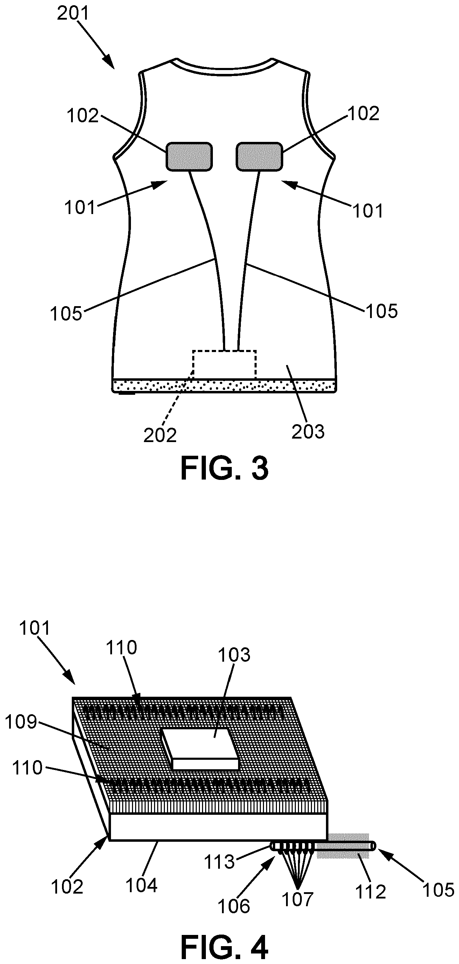

Different embodiments of the flexible electrode 102 are shown in figures 4, 5 and 7 - 10. The flexible electrode 102 is flexible to ensure that it can match the shape of the skin of the user to which it is applied. It may have a Shore A hardness in a range of 55 to 67.

In a non-limiting embodiment, the wearable article may be a brassiere that includes sensors to detect physiological signals, such as for example signals related to heart activity, breathing or body temperature of a wearer.

In alternative embodiment, the wearable article may be a garment comprising a clothing material. The clothing material may cover a portion of a body of the user. The garment may refer to an item of clothing or apparel. The garment may be a top. The top may be a shirt, t-shirt, blouse, sweater, dress, underwear, athletic clothing, swimwear, belt, jacket/coat, or vest.

In some embodiments, the flexible electrode 102 is made of material comprising silicone or essentially consisting of silicone. Silicone is suitable since it has low toxicity, high thermal stability and low chemical reactivity. Furthermore, it is flexible, withstands low and high temperatures and forms tight seals. It repeals water, which is advantageous to avoid influence of the user's sweat on measurements. In addition, silicone is cheap and easily available. A silicone electrode is robust and provides high wearing comfort for a user. Alternatively, natural rubber or other elastomers can be used as a material for the flexible electrode 102.

Different kinds of conductive particles may be added to the material of the flexible electrode 102 to influence the electrical properties of the flexible electrode 102, such as copper, iron, aluminium or graphite. Graphite is an appropriate material since it is highly conductive which makes it adapted for use in electronic products. Furthermore, it is non-toxic. Graphite is low-priced and easily available.

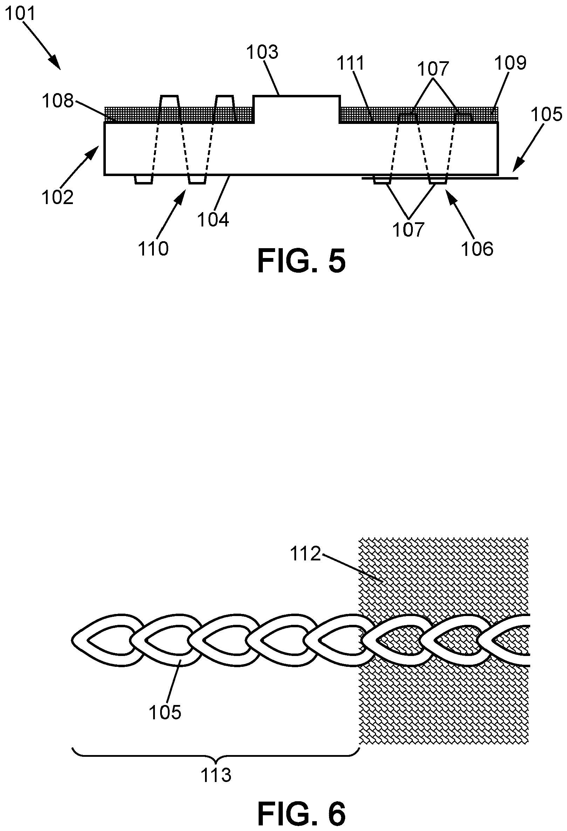

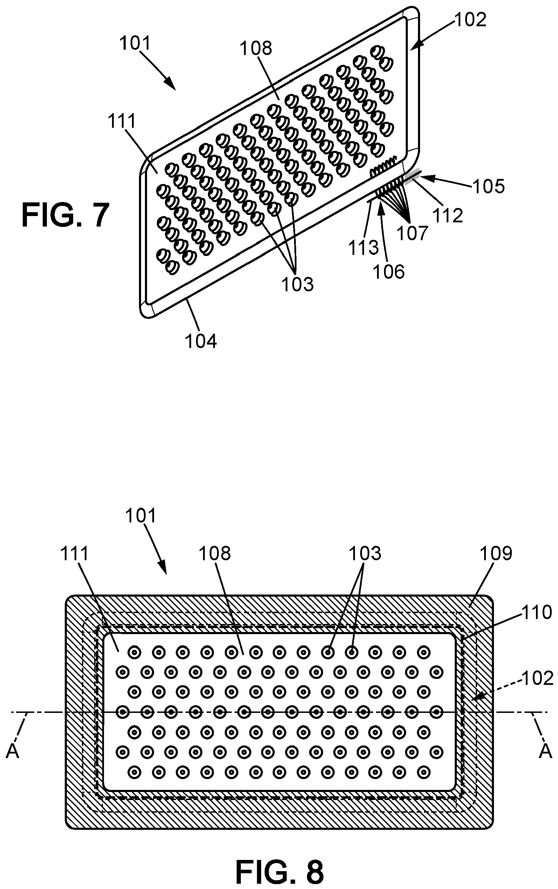

The flexible electrode 102 comprises one or more contact areas 103. Figures 4 and 5 show an embodiment of a flexible electrode 102 with one contact area 103. Figures 7-10 show embodiments of a flexible electrode 102 with a plurality of contact areas 103. Each contact area 103 according to the invention is integral with the body of the flexible electrode 102. In some embodiments, each contact area 103 comprises a protrusion protruding from the front face 108 of flexible electrode 102. Each protrusion may be column or square-shaped.

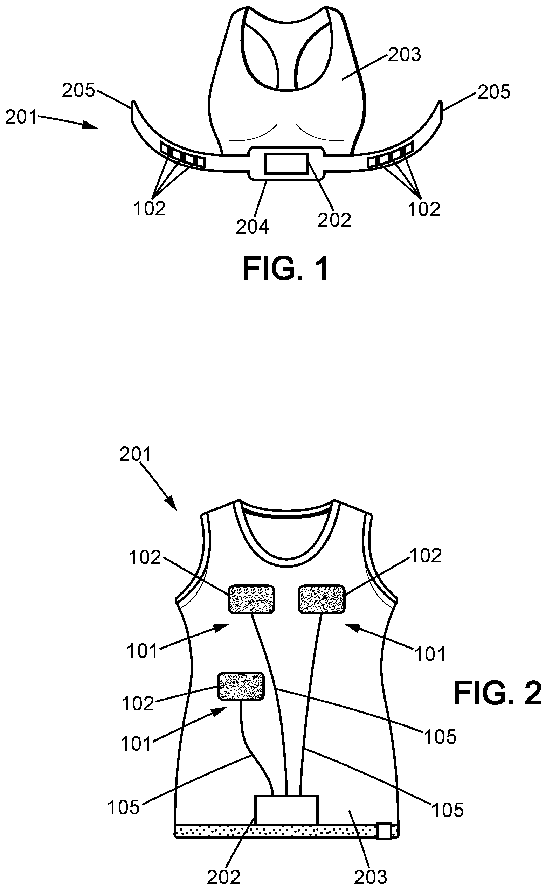

In the example of figure 1, the wearable article 201 is a brassiere that comprises several flexible electrodes 102 according to the invention. Flexible electrodes are thin, flexible, and easy to adjust to the contours of the body and stay at the place for long periods.

The brassiere may comprise a flexible support 203 covering parts of the chest and the back of a wearer, and further the shoulders in the form of shoulder straps. The flexible support 203 may be made of non-conductive fabric, like cotton/elastane or polyamide/elastane. The flexible support 203 may contain between 6% and 12% of elastane such that the wearable article 201 tightly fits the wearer's body. This makes sure that the sensors/flexible electrodes 102 included in the wearable article 201 are in close contact with the wearer's skin.

The brassiere may further comprise a waterproof housing 204 that receives an electronic board 202 configured to collect and process signals obtained from the flexible electrodes 102. The electronic board 202 may be coupled to a battery. The conductive yarn 105 is not shown in figure 1 for clarity reasons.

The waterproof housing 204 may be made from polyester plates that are soldered to each other on their respective edges. According to special embodiment, the waterproof housing 204 may be as disclosed in WO2021028223.

The waterproof housing 204 may be connected to two elastic belts 205 that are attached to opposite sides of the waterproof housing 204. When the brassiere is worn, the waterproof housing 204 may be located at the sternum of the wearer, and the elastic belts 205 may extend around a part of the torso of the wearer. The elastic belts 205 may be linked to each other at the back of the wearer.

The flexible support 203 of the brassiere may be connected to the waterproof housing 204. Therefore, a bottom portion of the flexible support 203 of the brassiere may be folded and sewn in order to create a pocket (not shown in figure 1) that receives the waterproof housing 204. A portion of the elastic belts 205 may protrude from the pocket in opposite directions.

The flexible electrodes 102 may be mounted at any suitable position on the inner side of the elastic belts 205, i.e. on the side of the elastic belts 205 intended to be in contact with the skin of the wearer. When tightened to the torso of the wearer, the elastic belts 205 press the flexible electrodes 102 against the torso.

Orifices may be created in the flexible support 203 of the brassiere. Contact areas 103 of the flexible electrodes 102 intended to be in contact with the skin of the wearer may protrude through the orifices.

The flexible electrodes 102 may be connected to the electronic board 202 by conductive yarn 105. To this effect, the electronic board 202 may comprise a conductive edge portion with holes to which the conductive yarn 105 is connected, for example is sewn.

Since the flexible electrodes 102 are located on the elastic belts 205 rather than at other positions in the brassiere such as the shoulder area, distances between the flexible electrodes 102 and the electronic board 202 are short, and short conductive yarns 105 may be used for connection of the flexible electrodes 102 and the electronic board 202.

The electrical connection path from the flexible electrodes 102 to the electronic board 202 may have a waterproof encapsulation.

According to this embodiment, the conductive yarn 105 may, at least partially, be encapsulated by elastic tape attached to the conductive yarn 105 by heat bonding. According to preferred embodiment, the conductive yarn 105 comprises a connection portion 113 that is not encapsulated by the elastic tape.

The electronic board 202 and a portion of the conductive yarn 105 connected to the electronic board 202 may be encapsulated by a silicone matrix in order to avoid intrusion of water into the electronic board 202. The encapsulation by a silicone matrix provides an additional protection for the electronic board 202, in addition to the placing of the electronic board 202 in the waterproof housing 204.

The encapsulated conductive yarn 105 may pass from the electronic board 202 through small orifices in the polyester plates of the waterproof housing 204 and further to the flexible electrodes 102. The orifices in the polyester plates may be sealed by elastic tape attached to the polyester plates by heat bonding, in order to ensure that the space enclosed by the polyester plates is waterproof.

Each flexible electrode 102 may be assembled with the conductive yarn 105, and then be placed in a waterproof encapsulation from which the contact area 103 of the flexible electrode 102 protrudes.

In another non-limiting embodiment shown in figures 2 and 3, the wearable article 201 is a T-Shirt that includes sensors (e.g. electrodes) to detect signals related to heart activity, breathing or body temperature of a wearer. In particular, the T-Shirt may comprise several flexible electrodes 102.

In some embodiments, particularly when the wearable article 201 is a T-Shirt, the flexible electrode 102 comprises more than one contact area 103 (not shown in figures 2 and 3 for clarity reasons), wherein each contact area 103 may comprise a protrusion, to be applied to a body of the user. Preferably the flexible electrode 102 comprises from 2 to 200, more precisely from 10 to 100 protrusions. In some embodiments, the contact areas 103 have intervals in a range of about 2 to about 8 mm therebetween.

The T-Shirt may comprise a flexible support 203 as above described covering the chest, back, abdomen and upper arms of a wearer. The T-Shirt may further comprise an electronic board 202 configured to collect and process signals obtained from the flexible electrodes 102. The electronic board 202 may be encapsulated by a silicone matrix in order to avoid intrusion of water into the electronic board 202. In addition, each flexible electrode 102 may have a waterproof encapsulation.

The flexible electrodes 102 may be located at any suitable position on the inner side of the T-Shirt, i.e. on the side of the T-Shirt oriented towards the skin of a wearer. The flexible support 203 of the T-Shirt may have elasticity and press the flexible electrodes 102 against the skin of a wearer.

The flexible electrodes 102 may be connected to the electronic board 202 by conductive yarn 105 attached to the inner side of the T-Shirt. Therefore, the electronic board 202 may comprise a conductive edge portion with holes to which the conductive yarn 105 is connected, for example is sewn. The conductive yarn 105 may have a waterproof encapsulation. The electrode assembly 101 of the flexible electrodes 102 and the conductive yarn 105 as well as the properties of the flexible electrodes 102 and the conductive yarn 105 are explained in relation to figures 4, 5 and 7-10.

Figures 4 and 5 show an electrode assembly 101 according to one embodiment of the invention. Flexible electrode 102 has contact area 103 intended to be applied to the body of a wearer of the wearable article 201, e.g. the brassiere or the T-Shirt. The contact area 103 is part of a front face 108 of the flexible electrode 102 and protrudes from the front face 108. The rest of the front face 108 that surrounds the contact area 103 is referred to as border area 111. The face of the flexible electrode 102 opposite to the front face 108 is referred to as back face 104.

Figure 7 is a schematic representation of an electrode assembly 101 according to another embodiment of the invention. Flexible electrode 102 has a plurality of contact areas 103 intended to be applied to the body of a wearer of the wearable article 201. The contact areas 103 are part of a front face 108 of the flexible electrode 102 and protrude from the front face 108. The rest of the front face 108 that surrounds the contact area 103 is referred to as border area 111.

Flexible electrode 102 may comprise a body of flexible material having embedded conductive particles. For example, the body is made of silicone and the conductive particles are carbon particles. Due to its flexibility such a flexible electrode adapts to the shape of the wearer's body while the wearer moves.

Connection between the flexible electrode 102 and the conductive yarn 105 is obtained thanks to first yarn 106 sewn in straight stitches through the front face 108 and the back face 104 of the flexible electrode 102 in order to create loops 107 on both faces. A portion of the conductive yarn 105 referred to as connection portion 113 may then be inserted into at least one, preferably at least two of the loops 107 located on back face 104.

In some embodiments, the first yarn 106 is a polyester yarn. According to preferred embodiment, the first yarn 106 is a conductive yarn 105. In particular embodiment the first yarn 106 is a conductive yarn 105 identical to conductive yarn 105.

During manufacturing process, loops 107 of the first yarn 106 may first be loose in order to allow insertion of the connection portion 113 of the conductive yarn 105 and may then be tightened in order to press the connection portion 113 against the back face 104 of the flexible electrode 102. The conductive yarn 105 is thus tied to the flexible electrode 102 and cannot slip out of the loops 107.

During manufacturing process, the elastic tape that may encapsulate the conductive yarn 105 may be removed from the connection portion 113 in order to create an electrical connection between the conductive yarn 105 and the flexible electrode 102.

According to one embodiment, the conductive yarn 105 may be sewn in chain stitches fixed to a flexible substrate 112 such as tulle (Figure 6).

An arrangement of chain stitches may extend beyond the flexible substrate 112. This arrangement of chain stitches extending beyond the flexible substrate 112 may form the above-mentioned connection portion 113 of the conductive yarn 105 and may be inserted into the loops 107.

Compared to single conductive yarn 105, the arrangement of chain stitches is more resilient and connection to the back face 104 of the flexible electrode 102 is improved.

In some embodiments, the chain stitches are replaced by another type of connector, such as for example FPC (flexible printed circuit) or perforated conductive element.

In some embodiments, the flexible electrode 102 may have a protective fabric 109, made for example from polyester, placed on its border area 111 and around the contact area 103. A second yarn 110 may be sewn through the protective fabric 109 and through the body of the flexible electrode 102 in order to attach the protective fabric 109 to the border area 111. This second yarn 110 may be part of the same yarn as the first yarn 106. The protective fabric 109 is intended to protect the border area 111 of the flexible electrode 102.

According to special embodiment shown in figure 7, the flexible electrode 102 comprises more than one contact area 103. In a particular case shown in figures 8 - 10, the protective fabric 109 may be placed over the periphery of the flexible electrode 102, the protective fabric 109 overlapping only the border area 111 localized at the electrode edges, and preferably extending beyond thereof. Second 110 and optionally first yarn 106 may be sewn through the protective fabric 109 and through the body of the flexible electrode 102 in order to attach the protective fabric 109 to the border area 111 located peripheral. Figure 8 shows a top-view of an electrode assembly 101 in which the protective fabric 109 is sewn to the flexible electrode 102 by the second yarn 110.

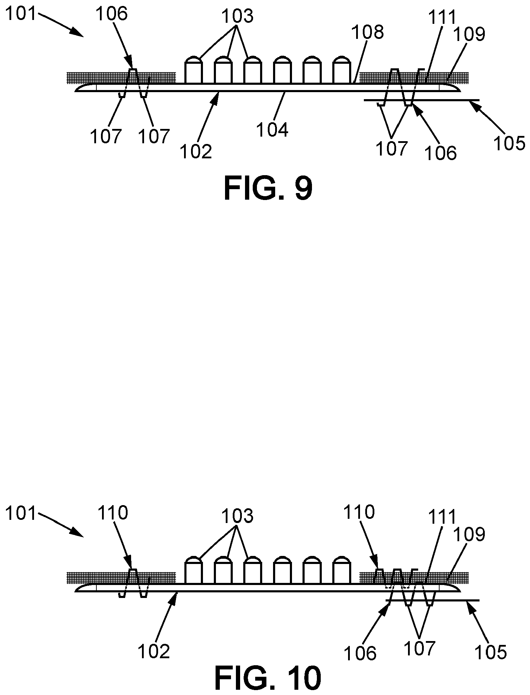

In the embodiment shown in Figure 9, connection between the flexible electrode 102 and the conductive yarn 105 may be obtained locally thanks to first yarn 106 sewn in straight stitches through the protective fabric 109 and through the front face 108 and the back face 104 of the flexible electrode 102 as described above. According to alternative embodiment shown in figure 10, during manufacturing process, in first step, the protective fabric 109 is positioned and sewn over the full electrode peripheral edges with the second yarn 110, and in second step connection between the flexible electrode 102 and the conductive yarn 105 may be obtained locally thanks to first yarn 106 sewn in straight stitches through the front face 108 and the back face 104 of the flexible electrode 102 without stitching through the protective fabric 109.

According to some embodiments, when the electrode assembly 101 is in place on the wearable article 201, the external edges of the protective fabric 109 may be, at least partially, attached to the flexible support 203 by heat bonding, which creates a waterproof encapsulation.

In order to decrease the impact of electromagnetic perturbations coming for example from electronic devices located in the vicinity of the flexible electrode 102 during measurements with the flexible electrode 102, an electromagnetic shielding material may cover intended locations of the flexible electrode 102. The electromagnetic shielding material may be made from conductive fabric.

The conductive fabric may be located in an area of the back face 104 of each flexible electrode 102.

Direct interaction between the conductive fabric and the flexible electrode 102 may be prohibited by placing an isolating material between the conductive fabric and the back face 104 of the flexible electrode 102. The conductive fabric may further be covered by a protective material, in order to avoid degradation of the conductive fabric, that may occur due to detachment of fibers of the conductive fabric.

When an electrocardiogram of the wearer is measured by two or more flexible electrodes 102 in the brassiere or in the T-Shirt, the conductive fabric may be connected to an additional electrode in the brassiere /the T-Shirt that establishes a ground potential. The ground potential may serve as a reference for the measurements with the two or more flexible electrodes 102. The conductive fabric may be connected to the additional electrode by conductive yarn 105 that is sewn to the conductive fabric.

It will be appreciated that the embodiments described above are illustrative of the invention disclosed herein and that various modifications can be made without departing from the scope as defined in the appended claims.

## Claims

1. An electrode assembly (101), comprising:
- a flexible electrode (102) having a front face (108) and a back face (104), the front face (108) comprising at least one contact area (103) to be applied to a body of a user and a border area (111) surrounding the at least one contact area (103);
- a first yarn (106) sewn in the border area (111) of the flexible electrode (102) and forming loops (107) on at least the back face (104); and
- a conductive yarn (105) passing through at least one of the loops (107) on the back face (104) and maintained on the flexible electrode (102) by the first yarn (106).

2. The electrode assembly of claim 1, wherein the at least one contact area (103) protrudes from the border area (111) on the front face (108).

3. The electrode assembly of claim 1 or claim 2, further comprising a protective fabric (109) disposed on the border area (111) of the front face (108) and exposing the at least one contact area (103).

4. The electrode assembly of claim 3, further comprising a second yarn (110) sewn through the protective fabric (109) and the border area (111) of the front face (108).

5. The electrode assembly of any one of the preceding claims, wherein the conductive yarn (105) comprises an arrangement of chain stitches.

6. The electrode assembly of any one of the preceding claims, wherein the conductive yarn (105) is sewn to a flexible substrate (112).

7. The electrode assembly of claim 6, wherein the conductive yarn (105) has a connection portion (113) extending beyond the flexible substrate (112), the connection portion (113) passing through at least some of the loops (107) and being maintained on the flexible electrode (102) by the first yarn (106).

8. A wearable article (201), comprising:
- an electronic board (202);
- a flexible electrode (102) and a conductive yarn (105) forming an electrode assembly (101) as claimed in any one of the preceding claims, wherein the conductive yarn (105) is further connected to the electronic board (202);
wherein the wearable article (201) is configured to press the at least one contact area (103) of the flexible electrode (102) against the body of the user wearing the wearable article (201).

9. The wearable article of claim 8, further comprising an electromagnetic shielding material located at the back face (104) of the flexible electrode (102), wherein an insulating material is placed between the back face (104) of the flexible electrode (102) and the electromagnetic shielding material.

10. The wearable article of any one of claims 8 and 9, further comprising an elastic belt (205) on which the flexible electrode (102) is mounted;
wherein the elastic belt (205) is configured to press the at least one contact area (103) of the flexible electrode (102) against the body of the user wearing the wearable article (201).

11. The wearable article of claim 10, further comprising a waterproof housing (204) configured to receive the electronic board (202), wherein the elastic belt (205) is attached to the waterproof housing (204).

12. The wearable article of claim 11, in the form of a garment, further comprising a flexible support (203) connected to the waterproof housing (204).

13. The wearable article of any one of claims 11 and 12, wherein the waterproof housing (204) comprises an orifice, and wherein the conductive yarn (105) maintained on the flexible electrode (102) and connected to the electronic board (202) passes through said orifice.

14. The wearable article of claim 13, wherein the orifice is sealed by elastic tape.

15. The wearable article of claim 8, further comprising a T-Shirt having a flexible support (203) configured to press the at least one contact area (103) of the flexible electrode (102) against the body of the user wearing the wearable article (201).
